# EUROPÄISCHE PATENTSCHRIFT

(11) **EP 0 603 871 B1**
(45) Veröffentlichungstag und Bekanntmachung des Hinweises auf die Patenterteilung: **11.03.1998**
(21) Anmeldenummer: 93120738.5
(22) Anmeldetag: 22.12.1993
(51) Int. Cl.: A61F 2/46, B01F 13/00, B05C 17/015

(54) **Verfahren und Vorrichtung zum Anmischen und Applizieren von Knochenzement**
Process and apparatus for mixing and applying bone cement
Procédé et dispositif pour mélanger et appliquer du ciment à os

(30) Priorität: 23.12.1992 DE 4243877
(43) Veröffentlichungstag der Anmeldung: 29.06.1994
(73) Patentinhaber: Jansson, Volkmar, Dr., D-82205 Gilching (DE); Zimmer, Markus, Dr., 83666 Waakirchen (DE)
(72) Erfinder: Jansson, Volkmar, Dr., D-82205 Gilching (DE); Zimmer, Markus, Dr., 83666 Waakirchen (DE)
(74) Vertreter: Haft, von Puttkamer, Berngruber, Czybulka

(56) Entgegenhaltungen:
- EP-A- 0 170 120
- EP-A- 0 380 867
- WO-A-87/05492
- WO-A-90/13264
- DE-A- 2 801 706
- DE-A- 3 640 279
- DE-A- 4 022 986
- US-A- 4 546 767

## Beschreibung

Die Erfindung betrifft ein Verfahren und eine Vorrichtung zum Anmischen und zur Verarbeitung von Knochenzement für medizinische Anwendungen gemäß Anspruch 1 bzw. 3.

"Knochenzement" wird in der Medizin in verschiedenen Bereichen benutzt. Ursprünglich wurde der auf Methylmethacrylatbasis bestehende Zement in der Gesichts- und Schädelchirurgie, später dann auch in der Zahnheilkunde eingesetzt. In der zementierten Endoprothetik (künstliche Gelenke wie Hüft- und Kniegelenk) werden die Implantate seit 1960 (Charnley J (1960) Anchorage of the femoral head prosthesis to the shaft of the femur. J Bone Joint Surg 42-B:28) mit Hilfe von Knochenzement im Knochen verankert. Speziell bei der Verankerung der mechanisch hoch belasteten Endoprothesen kommt der Qualität dieses Knochenzementmantels bei der langfristig stabilen Verankerung der Implantate im Knochen eine entscheidende Bedeutung zu (Gruen TA, McNeice MS, Amstutz HC (1979) "Modes of failure" of cemented stem-type femoral components. Clin Orthop Rel Res 141:17-27). Eine schlechte Knochenzementqualität führt demnach frühzeitig zu einer Zementzerrüttung und damit zu einer vorzeitigen Implantatlockerung.

Dabei ist insbesondere die komplette Zementfüllung des Markraumes, die möglichst verunreinigungsfreie (z.B. Blut, Spülflüssigkeit, Markraumfett, Luft) Zementeinbringung zwischen Implantat und Knochen sowie das möglichst blasenfreie Anmischen des Knochenzementes (Draenert K (1988) Forschung und Fortbildung in der Chirurgie des Bewegungsapparates 2. Zur Praxis der Zementverankerung. Art and Sience, München) wichtig.

Da der Knochenzement von Haus aus nur über geringe Dauerschwingfestigkeiten (Willert HG, Buchhorn G (1987) Knochenzement: Werkstoff, klinische Erfahrungen, Weiterentwicklungen. Aktuelle Probleme in Chirurgie und Orthopädie. Bd. 31. Huber Verlag Bern, Stuttgart, Toronto) im Vergleich zu den Endoprothesenwerkstoffen (z.B. Titanschmiedelegierungen, Chrom/Cobalt-Legierungen) verfügt, muß daher bei der Zubereitung des Knochenzementes darauf geachtet werden, Lufteinschlüsse (Blasen) zu vermeiden, da diese im Sinne von Kerben die Festigkeit des Knochenzementes weiter herabsetzen (DRAENERT, s.o.).

Im Spezialfall der Hüftendoprothetik unterscheidet man im Fall des coxalen Femurs (Hüftschaft) prinzipiell zwei Arten der Zementeinbringung: Bei der primären Zementeinbringung wird zuerst der Knochenzement in die Markraumhöhle verbracht, danach wird die Prothese in diesen hineingeschoben. Bei der sekundären Zementiertechnik wird dagegen zuerst die Prothese in die Markhöhle gelegt, danach wird der Knochenzement in den verbleibenden Zwischenraum gedrückt, wobei dieses im Fall der Zementkanalprothese (Jansson V, Kühne J-H, Zimmer M (1992) Die Zement- und Saugkanalprothese - Funktionsprinzip und Ergebnisse experimenteller Untersuchungen eines alternativen Zementierkonzeptes. Orthopädische Praxis. 7:470-473) durch ein in der Prothese befindliches Bohrlochsystem bei gleichzeitiger Entlüftung des Markraumes durch ein ebenfalls in der Prothese befindliches Bohrkanalsystem geschieht.

Optimalerweise wird letztendlich ein kompletter Zementmantel um die Prothese mit definierter Eindringtiefe in die Spongiosa benötigt, um die Belastung an der Grenzfläche Zement/Knochen zu minimieren. Nach JANSSON (Jansson V, Heimkes B, Zimmer M (1993) Stress transfer at the femoral bone/bone cement interface as a function of the cement thickness. Arch Orthop Trauma Surg. 111:in press) liegt dabei die idealerweise anzustrebende Eindringtiefe des Knochenzementes in die Spongiosa bei 2 - 3 mm. Am reproduzierbarsten läßt sich diese Forderung zur Zeit mit der Zementkanalprothese (s.o.) realisieren.

Bei der Einbringung von Knochenzement speziell in das coxale Femurende besteht die Gefahr von Lungenembolien, da es in Abhängigkeit vom intramedullär herrschenden Druck zu einer Einschwemmung von Fett und Knochenmark in die Blutbahn kommt (Wenda K, Ritter G, Ahlers J, Issendorf WD (1990) Nachweis und Effekte von Knochenmarkseinschwemmungen bei Operationen im Bereich der Femurhöhle. Unfallchirurg 93:56-61). Aus diesem Grund sollte der bei der Implantation der Prothesen intramedullär herrschende Druck bzw. das dabei aus der Spongiosa in die Blutbahn verdrängte Volumen möglichst klein gehalten werden.

Üblicherweise wird der Zement wie folgt angemischt: In eine Schale wird das Monomer (Flüssigkeit) geschüttet, danach das Polymer (Pulver) hinzugetan. Beide Komponenten haben Raumtemperatur. Die Komponenten werden dann mit Hilfe eines Spatels gemischt und der Zement in das Op-Gebiet verbracht. Dieses kann nach Umfüllen des Zementes in eine Zementspritze oder aber durch digitales Hineinstopfen des Zementes in die entsprechende Körperhöhle geschehen. Lufteinschlüsse sind bei dieser Art der Verarbeitung des zähen Zementes nicht zu umgehen.

Eine einfache Methode, Lufteinschlüsse während der Zubereitung (das ist der während der Operation stattfindende Mischvorgang zwischen der flüssigen Monomer- und der pulverförmigen Polymerkomponente) des Knochenzementes zu reduzieren, besteht darin, den Knochenzement unter Unterdruckbedingungen ("Vacuum"verfahren) anzumischen (DRAENERT, s.o.).

Bei den dazu üblichen Verfahren (z.B. DE-A1-3640279, Draenert s.o., DE-A1-3425 566, DE-A1-37 08 442 A1, DE-A1-40 22 985 ) wird an das Mischgefäß, in dem die Komponenten miteinander verrührt werden, ein Schlauch angeschlossen, der über eine Pumpe einen Unterdruck in dem Mischgefäß erzeugt.

Nachteilig bei einem solchen Verfahren ist zum einen der apparativ relativ hohe Aufwand (steril zu betreibenede Unterdruckpumpe, Entsorgung des abgesaugten Luft/Monomergemisches über entsprechende Filter, Umfüllen des Zementes aus dem Mischbecher in die Spritzenkartusche, Aufsetzen der Spritzenkartusche auf die ebenfalls steril zu handhabende Spritzpistole), zum anderen jedoch auch die Tatsache, daß ein Teil der Monomerkomponente bei Anlegen eines Unterdruckes verdampft und abgesaugt wird und sich so das Mischungsverhältnis der zwei Komponenten ändert (Herstellerangaben, s. Brief Haereus/Kulzer vom 3.12.92). Speziell aus diesem Grund soll das Anmischen des Knochenzementes bei niedrigen Temperaturen (ca. 4°C) erfolgen, da bei niedrigen Temperaturen die Menge der verdampften Monomerflüssigkeit dem niedrigeren Dampfdruck entsprechend geringer ist. Die niedrige Zementtemperatur beim Mischvorgang hat außerdem den Vorteil, daß dadurch die Viskosität des Komonentengemisches gesenkt wird, so daß Luftblasen leichter nach oben entweichen und aus dem Zement austreten können.

Insbesondere die Anmischung des Zementes mit einem offenen Vakuum - also einer kontiniuerlichen Absaugung - stellt ein erhebliches Problem dar: Nur bei exakt eingehaltener Anmischtemperatur des Zementes von 4°C bleibt der "weggesaugte" Monomeranteil so gering (s.o.), daß mit keinen wesentlichen Einbußen der Zementfestigkeit gerechnet werden muß. Bei höheren Temperaturen können jedoch erhelbliche Mißverhältnisse der Komponenten mit entsprechenden Folgen für die Zementfestigkeit die Folge sein. Derartige Temperaturerhöhungen können sich zum Beispiel durch Kühlschranktemperaturschwankungen, in denen der Knochenzement gelagert wird, oder aber durch ein zu frühes Herausnehmen des Zementes aus dem Kühlschrank bei der Operation oder aber durch eine operationsbedingte Verzögerung des Zementiervorganges ergeben.

Ein weiterer Nachteil der offenen Vakuumtechnik ist, daß die entstehenden Monomerdämpfe entsorgt werden müssen, was nur mit Hilfe entsprechender Filter zu bewerkstelligen ist.

Aufgrund der Kompliziertheit dieser Verfahren mit der allein daraus resultierenden großen Zahl von Fehlerquellen sowie der geringen Fehlertoleranz in Bezug auf die Verarbeitungstemperatur des Zementes konnte sich die an sich wünschenswerte Vakuumanmischung von Knochenzement in der Endoprothetik nicht durchsetzen. Dazu kommt, daß durch den hohen apparativen Aufwand und die Verwendung einer Reihe von Einmalartikeln dieses Verfahren sehr teuer wird.

Ein weiteres wesentliches Problem bei der Implantation speziell am coxalen Femurende (Hüftschaft) stellt die intraoperative Druckerhöhung im Markraum mit entsprechenden Volumenverschiebungen von Blut und Markraumfett in die Blutbahn dar. Wie oben dargelegt (Wenda... s.o.), können derartige Druckerhöhungen zu ernsthaften intraoperativen Komplikationen führen. Andererseits ist jedoch eine gewisse Druckerhöhung bei der Implantation nicht zu umgehen, wenn eine stabile Verankerung des Implantates erreicht werden soll. Daraus folgt, daß die Zementapplikation in das coxale Femur so druckgesteuert erfolgen muß, daß einerseits die druckbedingte Volumenverschiebung von Blut und Markraumfett möglichst gering ist, andererseits die erforderliche Zementpenetration in die Spongiosa von 2 - 3 mm (Jansson... s.o.) erreicht wird.

Dabei muß die Implantation in primärer Zementiertechnik von der sekundären Zementiertechnik unterschieden werden.

Bei der primären Zementiertechnik sollte das Markraumvolumen mit einem zunächst kleinen Druck (ca. 0.2 - 0.8 bar) aufgefüllt werden. Da der Femurschaft individuell ein sehr unterschiedliches Volumen aufweist, muß in dieser Füllphase ein bestimmter Druck bis zum Ende der Füllphase aufrecht erhalten werden. Ein distaler Markraumstopper sollte zur Begrenzung des Markraumes nach distal verwendet, ein Absaugschlauch zur Entlüftung des Femurs eingelegt werden. Nach kompletter Füllung des Markraumes sollte dann durch Nachpressen eines bestimmten Zementvolumens die gewünschte Knochenzementpenetration in die Spongiosa von 2 - 3 mm erzwungen werden. Geht man beispielhaft von einem durchschnittlichen Prothesendurchmesser im proximalen (spongiösen) Bereich von 2.5 cm und einem spongiösen Bereich des Femurs von etwa 4 cm Länge aus, so ergäbe sich ein Volumen von 6.3 ml das in die Spongiosa gedrückt werden müßte, um eine Knochenzementpenetration von 2 mm zu erhalten. Große Femurschäfte mit weiter nach distal reichender Spongiosierung benötigen ein entsprechendes größeres Zementvolumen. Werden aus besonderen klinischen Gründen tiefere Zementpenetrationen gewünscht, so muß ein entsprechendes größeres Volumen in die Spongisa gedrückt werden, bei geringerer Eindringtiefe ein entsprechend geringeres Volumen.

Bei der sekundären Zementiertechnik im Beispiel der Zementkanalprothese ist die druck- und volumengesteuerte Zementapplikation bei der Implantation einer Zementkanalprothese für die korrekte Implantion besonders wichtig, da durch den speziellen Druckverlauf bei der Implantation die gewünschte Zementpenetrationstiefe in die Spongiosa von 2 - 3 mm erreicht werden kann. Dabei wird die Totraumvolumenfüllung (Totraumvolumen: Volumen des in der Prothese befindlichen Zementkanalsystems und nur das Volumen des frei aus der Prothese austretenden Zementes, bevor der Zement die Markhöhle soweit füllt, daß es zu relevanten intramedullären Druckerhöhung kommt) mit einem hohen Druck, die Markhöhlenauffüllung mit einem niedrigeren Druck vorgenommen. Dieser sehr niedrige Druck kann bei der Zementkanalprothese bis zum Ende der Zementaushärtung aufrecht erhalten werden, so daß mit niedrigen Drucken eine tiefe Penetration des Zementes in die Spongiosa erzielt werden kann (Jansson... s.o.).

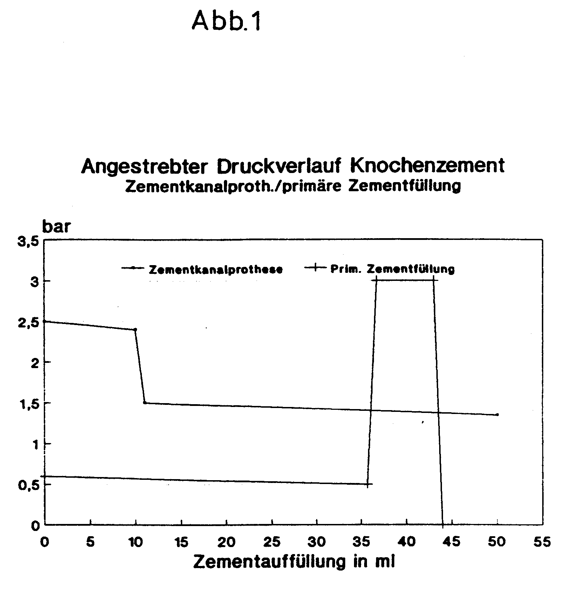

Abb.1 zeigt beispielhaft die prinzipiell erwünschte Druck/Volumen-Beziehung bei einer angenommenen Implantation für primäre Zementiertechnik bzw. für die Zementkanalprothese. Die angegeben Drucke und Volumina stellen in etwa den wissenschaftlichen Stand der Technik bei der Implantation eines Hüftendoprothesenschaftes dar, jedoch sind Änderungen der Druck/Volumen-Beziehung je nach wissenschaftlichem Erkenntnisstand möglich.

Nachteilig bei allen bisherigen Zementapplikationsystemen ist, daß keines dieser Systeme die volumen- und druckgesteuerte Zementapplikation ermöglicht. Zwar erlaubt das DRAENERT-System gemäß DE-A1-34 25 566 die Applikation des Zementes mit einem - per Knopfdruck und Augenmaß - in irgendeiner Weise eingestellten Zement- bzw. Implantationsdruck. Jedoch ist weder die exakte Höhe dieses Druckes noch die dabei applizierte Menge des Zementes meß- oder sogar steuerbar. Keines der vorhandenen Systeme verfügt über ein erforderliches Meß- und Steuersystem zur volumen- und druckgesteuerten Zementapplikation, und erst recht läßt sich bei keinem System eine Zementapplikation mit einer durch das System automatisch vorgegebenen solchen Volumen-Druckcharakteristik weder bei der primären noch bei der sekundären Zementiertechnik erzielen.

Wie in Abb.1 dargestellt, läßt sich bei der primären Zementiertechnik die forcierte Zementpenetration durch kurzfristige Druckerhöhung auf z.B. 3 bar erzielen, jedoch ist es sicher auch möglich, mit höherem oder niedrigerem Druck bei entsprechend kürzerer oder längerer Einwirkzeit den gleichen Effekt zu erzielen. Wichtig ist bei dieser Technik vor allem, daß das Volumen von ca. 6 ml in relativ kurzer Zeit (z.B. 10-20 s) appliziert wird, da bei der primären Zementiertechnik ja noch genügend Zeit zur Verfügung stehen muß, um die Prothese bei noch weichem Zement einzuschieben.

Aus der WO 90/13624 ist eine Vorrichtung zum Mischen von Knochenzement bekannt, die einen Mischbecher aufweist, der als Boden einen Kolben und an einem Ende eine Verschlußvorrichtung hat, mit der der Kolben an dem einen Ende des Mischbechers verriegelt und entriegelt werden kann. Außerdem ist ein Mischaufsatz vorgesehen, der auf das andere Ende des zylindrischen Mischbechers aufgesetzt werden kann und der über einen Mischstab zum Vermischen der Einzelkomponenten des Knochenzementes verfügt. Der Mischaufsatz ist gegenüber dem Mischbecher luftdicht abgedichtet; ebenso ist der in dem Mischaufsatz längsverschieblich gelagerte Mischstab gegenüber dem Mischaufsatz luftdicht abgedichtet. Auch dieser Mischbecher kann über einen Schlauch mit einer Vakuumquelle verbunden werden, so daß das Mischen ebenfalls unter Vakuum erfolgt.

Es ist Aufgabe der Erfindung, erstens ein apparativ einfaches Verfahren zur Anmischung von Knochenzement mit deutlich erhöhter Fehlertoleranz anzubieten und zweitens eine Zementapplikation zu ermöglichen, die eine durch das System vorgegebene automatisch druck- und volumengesteuerte Zementeinbringung in eine bestimmte Körperhöhle erlaubt. Insbesondere ist das Ziel:
1. Den Knochenzement in möglichst hoher Qualität (lufteinschlußarm) und einem korrekten Mischungsverhältnis der Komponenten zueinander in einem geschlossenen System anzumischen.
2. Die mit dem System vorzugebende automatisierte druck- und volumengesteuerte Zementapplikation sowohl bei Verwendung einer Zementkanalprothese als auch bei der primären Zementiertechnik zu ermöglichen und so
3. eine den operativen Bedürfnissen angepaßte Applikationstechnik mit einer durch das Verfahren bestimmten definierten und reproduzierbaren Zementpenetration in die Spongiosa zur Verfügung zu stellen.
4. Eine möglichst risikofreie Applikation des Zementes im Sinne der Vermeidung hoher intrafemoraler Implantationsdrucke zur Vermeidung von Embolien zu erzielen.

Zum Erreichen der oben geannnten Ziele muß das Knochenzementapplikationssystem deshalb folgende Bedingungen erfüllen:
1. Klare, einfache Arbeitsschritte in definierter Folge,
2. Erhöhung der Fehlertoleranz durch apparative Vereinfachung durch Wegfall der Unterdruckpumpe und Erzeugung des Unterdruckes in einem geschlossenen System, so daß kein Monomer - mit entsprechenden Auswirkungen auf das Mischungsvehältnis der Komponenten - abgesaugt werden kann, so daß das System in dieser Hinsicht im wesentlichen unabhängig von der Zementtemperatur ist.
3. Möglichkeit der durch das System vorzugebenden automatischen druck- und volumengesteuerten Zementeinbringung.
4. Modularer Aufbau, angepaßt an das vorhandene Endoprothesensystem, die technischen Einrichtungen im Operationssaal und die Ansprüche des Operateurs an die Zementqualität,
5. Größtmögliche Sicherheit für Patient und Op-Personal.

Die oben angeführten Aufgaben werden für ein Verfahren zum Mischen von Knochenzement im Vakuum durch die Merkmale des Patentanspruches 1 und für eine Vorrichtung durch die Merkmale des Patentanspruches 4 gelöst. Weitere Ausgestaltungen der Erfindung gehen aus den Unteransprüchen hervor.

Erfindungsmäßig wird demnach vorgeschlagen, daß
1. die Mischung der Komponenten in einem abgeschlossenen Vakuum erfolgt. Damit wird erstens ein korrektes Mischungsverhältnis der Komponenten auch bei höheren Temperaturen gewährleistet, zweitens (durch die Vakuumtechnik) die Blasenbildung verringert und drittens der Austritt schädlicher Gase in den Operationssaal reduziert.
2. bestimmte Volumina des Knochenzementes mit einem durch das Systen vorgegebenen bzw. vorzugenden Druck appliziert werden können. Dieses dient der definierten und reproduzierbaren Zementpenetration und der Begrenzung des Implantationsdruckes zum Schutz vor Lungenembolien. Dabei soll sowohl die zur Implantation bei der Zementkanalprothese (sekundäre Zementiertechnik) als auch die zur primären Zementiertechnik günstige Druck-Volumencharakteristik (Abb.1) realisiert werden können.
3. ein austauschbarer Applikationsaufsatz durch geeignete Wahl seiner Form das Einbringen von Zement in die vorgesehene Körperhöhle oder die spezielle Endoprothese gewährleistet.
4. der Zementbehälter leicht zu reinigen und der ausgehärtete Zement durch hinterschneidungsfreie Konstruktion des Mischbechers und der verschiedenen Aufsätze ausschlagbar ist.
5. der Behälter zum Mischen der Komponenten auch zur Applikation des Zementes verwendet wird. Damit wird ein Arbeitsschritt (Umfüllen des Zementes) und ein Gerät gespart, ein Vorteil, der bereits von RICHNER R (Richner R (1978) Auslegeschrift 28 01 706) beschrieben ist.
6. ein modulares System vorgesehen ist, das die sukzessive Erweiterung und Anpassung der Zementiertechnik an die jeweiligen intraoperativen Bedingungen und die verwendeten Implantate erlaubt, wobei das System vom herkömmlichen Anmischen ohne Vakuum bis hin zur Implantation einer Zementkanalprothese angepaßt werden kann.

In einer bevorzugten Ausführung besteht der Mischbecher aus einem Kolben (2) und einem Zylinder (1) von etwa 4 cm Durchmesser, der so ein Volumen von 210 ml faßt. Der Boden des Zylinders (1) ist von dem Kolben (2) verschlossen, der zu Beginn des Mischvorganges in dem Einrastverschluß (5) blockiert sein kann.

Außer dem Anmischen mit Hand mit Hilfe eines Spatels ohne Vakuum kann der Mischvorgang in diesem Zylinder vorzugsweise wie folgt durchgeführt werden: Zunächst werden die Knochenzementkomponenten in den Behälter bestehend aus Zylinder (1) und Kolben (2) eingefüllt. Durch Hochschieben des Kolbens (2) (z.B. durch Verwendung der Stempelstange (9)) erreicht der Zement an der Oberkante des Zylinders (1) eine Markierung, die so angebracht ist, daß dadurch zwischen dem Zementoberrand und der Oberkante des Zylinders noch soviel Volumen verbleibt, wie der Flügel des Mischstabes (4) einnimmt. Der Mischaufsatz (3) wird mit dem Überwurf (6) auf den Zylinder luftdicht aufgesetzt, wobei die Luftdichtigkeit entweder durch eine entsprechend genaue Fertigung oder aber durch Verwendung eines Dichtringes erreicht werden kann. Durch Herunterdrücken des Mischstabes (4) wird der Kolben (2) wieder nach unten gedrückt und rastet in dem Verschluß (5) ein. Durch geeignete Lagerung und Abdichtung (10) des Mischstabes (4) in dem Mischaufsatz (3) kann der Stab luftdicht in dem Zylinder längsverschoben und gedreht werden. Die Abdichtumg erfolgt z.B. mit einem O-Ring oder aber durch paßgenaue Fertigung der Lagerung (10) des Mischstabes in dem Mischaufsatz. In dem so entstandenen Mischbehälter herrscht jetzt ein Vakuum, das sich aus dem Gesamtvolumen des Zylinders und dem Volumen der im Zement eingeschlossenen Restluft ergibt. Werden z.B. zwei Zementportionen gleichzeitig angerührt (Ausgangsvolumen ca. 160 ml), so ergibt sich bei einem Restluftgehalt des noch nicht fertig gemischten Zementes von ca. 25% ein maximal erzielbares Vakuum von ca. 80% (0.2 bar). Bei der häufigeren Anmischung nur einer Zementportion läßt sich sogar ein noch höheres Vakuum erzielen.

Durch Drehen des Mischstabes werden die Zementkomponenten vermischt. Dabei kann der Mischstab in der Gleitlagerung (10) gedreht und nach oben und unten bewegt werden. Dabei sorgen an dem Mischstab angebrachte Flügel (mindestens jedoch ein Flügel) für eine Durchmischung des Zementes. Diese Flügel können eine propellerartige Anstellung besitzen, die in einer besonders bevorzugten Ausführung so geformt sind, daß bei Rotation die peripheren Anteile des Zementes nach unten und die zentralen Anteile nach oben gedrückt werden oder umgekehrt (je nach Drehrichtung des Mischstabes). Damit ist ein Aufsteigen der Luftblasen an die Oberfläche gewährleistet.

Es ist im übrigen auch möglich, den Mischstab vom Boden des Behälters durch den Kolben zu führen und mit dem Kolben verriegelbar auszugestalten, so daß entsprechend dr Zement gemischt und der Kolben verschoben werden kann. Der Behälter wird in diesem Falle durch einen abgeschlossenen Deckel verschlossen.

Nach dem Durchmischen des Zementes (ca. 1 - 2 min) wird der Verschluß (5) geöffnet und der Kolben kann nach oben gleiten. Der Mischaufsatz wird durch Lösen des Überwurfes abgenommen. Der Zement kann jetzt z.B. mit einem Spatel entnommen und weiter verarbeitet werden. Andererseits kann aber auch ein für die geplante Implantation geeigneter Zementapplikationsaufsatz (12) mit dem Überwurf an dem Zylinder befestigt werden. Mit Hilfe des Stempels (9) kann der Zement wie bei einer Spritze ausgedrückt werden, wobei der Stempel (9) genau in die Aussparung (11) des Kolbens (2) paßt.

Andererseits kann die Applikation des Zementes auch mit Hilfe des Druckapplikators (35) erfolgen, was insbesondere bei der Implantation im Bereich des coxalen Femurs (Hüftschaft) eine besonders vorteilhafte definierte Penetration des Knochenzementes in die Spongiosa zur Folge hat.

Dazu wird der mit fertig gemischtem Zement gefüllte und mit dem geeigneten Applikationsaufsatz (12) versehene Zylinder (1) zunächst mit dem Druckapplikator verbunden. In einer bevorzugten Ausführungsform wird dabei der Zylinder so weit in den Aufnahmezylinder (26) des Druckapplikators (35) eingeschoben, bis durch Druck der Kolbenstange (14) auf den Kolben (2) der Zement am Applikationsaufsatz (12) erscheint. In dieser Stellung wird der Zylinder (1) in dem Aufnahmezylinder (26) mit einer geeigneten Vorrichtung (27) fixiert, was z.B. mit Hilfe einer Flügelschraube geschehen kann.

Der Druckapplikator soll sowohl für die primäre als auch für die sekundäre Zementiertechnik (z.B. Zementkanalprothese) geeignet sein. Vorzugsweise soll dieses mit einem einzigen Druckapplikator möglich sein, der z.B. durch auswechselbare Druckminderer ensprechend angepaßt sein kann. Der Druckapplikator soll so konstruiert sein, daß ein Ausdrücken des Knochenzementes aus dem Zylinder (1) mit den in der Abb.1 dargestellten Druckverläufen möglich ist. Die in Abb.1 dargestellten Druck-/Volumenkurven zeigen beispielhaft den prinzipiellen Einspritzvorgang bei der Implantation eines Hüftschaftes. Änderungen dieser Druck-/Volumencharakteristik an die Bedürfnisse anderer Körperhöhlen, anderer Knochenzemente mit anderen Viskositätseigenschaften oder anderer Anforderungen an die Knochenzementpenetrationstiefe sind durch Änderungen z.B. der Zahl, der Position und der Druckcharakteristik der Druckminderer oder durch andere konstruktive Maßnahmen möglich.

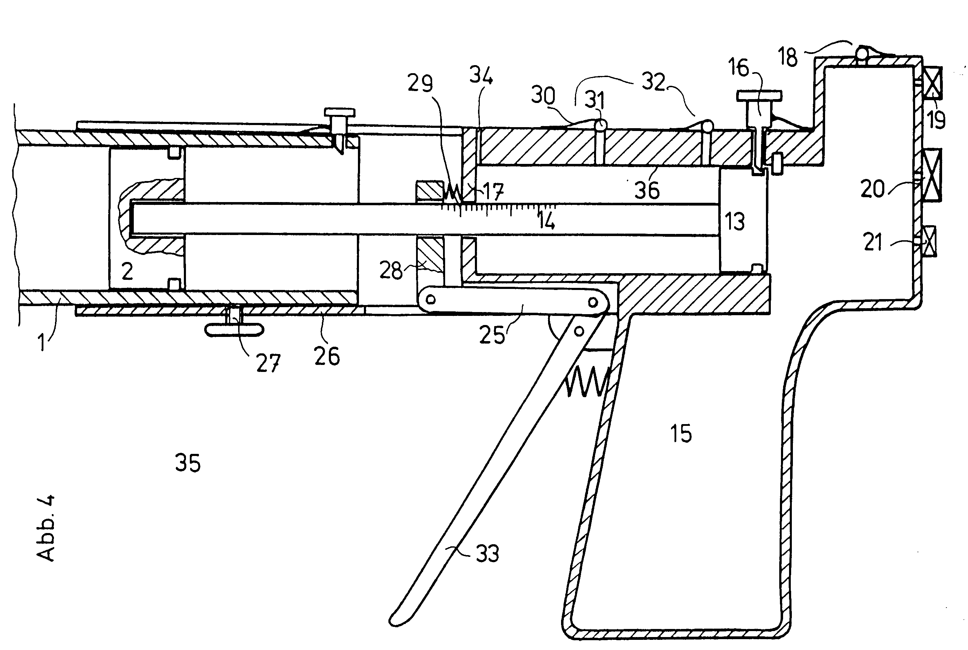

Abb.4 zeigt eine besonders bevorzugte Ausführung eines Druckapplikators. Durch Öffnen des Ventils (21) wird der Luftdruck hinter dem Kolben (13) abgelassen, so daß der Kolben in seine Ausgangsposition geschoben und mit der Feststellvorrichtung (16) in dieser Position blockiert werden kann. Über den Druckluftanschluß (20) wird das Luftreservoir (15) bis zu dem durch den Druckbegrenzer (18) vorgegebenen Druck gefüllt. Damit ist der auf den Kolben (13) und damit auf den Kolben (2) wirkende Initialdruck vorgegeben. Bei Implantation einer Zementkanalprothese wird jetzt der Applikationsaufsatz mit der Zementkanalprothese verbunden, wobei diese Verbindung z.B. durch Einführen und dadurch leichtes Verklemmen des leicht konischen Zementapplikatonsaufsatzes (12) in die Zementeinfüllbohrung der Zementkanalprothese erfolgen kann. Auch andere Verbindungen wie z.B. Bajonettverschlüsse oder Gewinde sind möglich. Durch Entriegelung des Verschlusses (16) wird der Kolben (13) nach vorne gedrückt und treibt über die Kolbenstange (14) und den Kolben (2) den Zement aus den Zylinder (1). Der Zementdruck wird aus dem Flächenverhältnis der Kolben (2) und (13) sowie dem Druck im Luftreservoir bestimmt. Eine Entlüftung (34) verhindert den Aufbau eines Gegendruckes zwischen Kolben (13) und Anschlagsperre (17). Hat der Kolben ein bestimmtes Volumen Zement aus dem Zylinder (1) herausgedrückt, dann fährt der Kolben (13) an einem Druckminderer (32) vorbei, der den Druck im Luftreservoir reduziert. Dadurch wird erreicht, daß das Totraumvolumen der Zementkanalprothese mit einem hohen Druck (s. Abb.1), das Markraumvolumen mit einem niedrigeren Druck gefüllt werden kann. Dieser Druck kann bis zur Aushärtung des Zementes aufrecht erhalten werden (ca. weitere 10 min), so daß mit einem niedrigen Druck eine ausreichende Zementpenetration erfolgen kann. Nach JANSSON (Jansson V, Kühne J-H, Zimmer M (1992) Die Zement- und Saugkanalprothese - Funktionsprinzip und Ergebnisse experimenteller Untersuchungen eines alternativen Zementierkonzeptes. Ortopädische Praxis. 7:470-473) reichen dabei für die Zementkanalprothese Drucke zwischen 0.5 - 1.5 bar aus. Diese niedrigen Drucke haben einen günstigen Einfluß auf die intraoperative Gefahr von Lungenembolien (Wenda K, Ritter G, Ahlers J, Issendorf WD (1990) Nachweis und Effekte von Knochenmarkseinschwemmungen bei Operationen im Bereich der Femurhöhle. Unfallchirurg 93:56-61). Sollten weitere Absenkungen oder aber eine stufenweise Reduzierung des Druckes gefordert werden, so kann dieses durch weitere Druckminderer (32) erreicht werden.

Die geforderte Druckreduzierung kann auch dadurch realisiert werden, daß Kolben (13), Kolbenstange (14) oder damit verbundene Teile beim Ausfahren des Kolbens (13) an Schaltern vorbeifahren, die, umgeschaltet, ihrerseits ein Druckminderungsventil im Luftdruckreservoir betätigen. Die Schaltung und Rückkopplung kann dabei auf mechanischem, hydraulischem oder elektrischem Weg erfolgen, auch ein lichtoptischer Schaltmechanismus ist denkbar.

Es ist auch möglich, den Druckapplikator ohne Luftreservoir auszuführen. Der Druckluftanschluß wird dann über einen Druckminderer ohne Zwischenschaltung eines Luftreservoirs mit direkter Wirkung auf den Kolben (13) an den Druckapplikator angeschlossen. Die geforderte Druckreduzierung wird dann durch den oben beschriebenen Schaltmechanismus realisiert, wobei die Rückkopplung jetzt direkt auf den Druckminderer zwischen Luftanschluß und Druckapplikator wirkt.

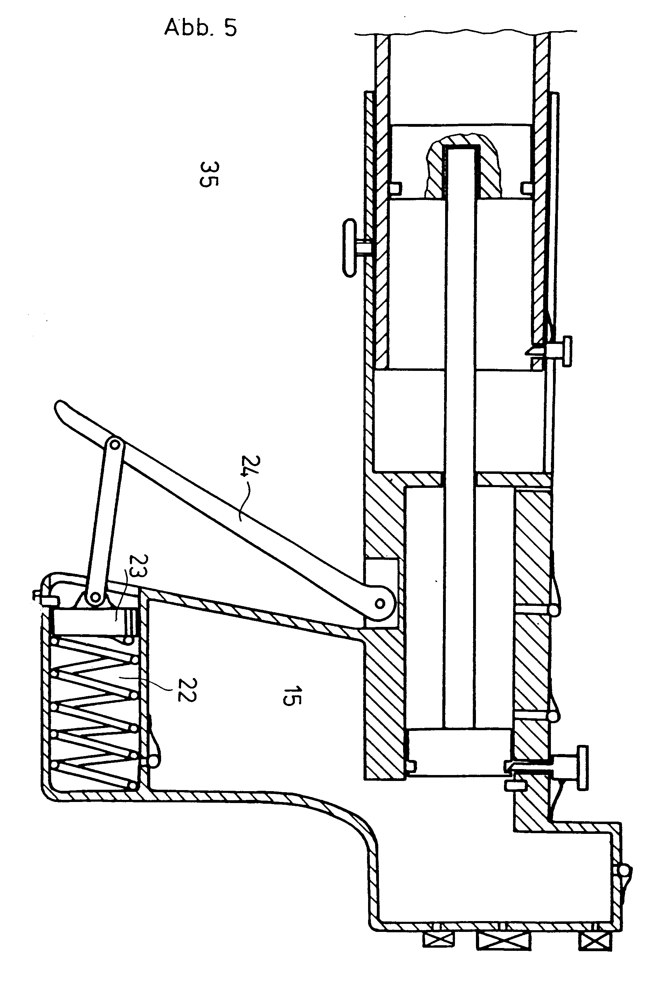

Es ist auch möglich, den geforderten Luftdruck im Luftreservoir mit Hilfe einer Handpumpe zu erzeugen, die wie in Abb.5 direkt in den Druckapplikator integriert oder aber vorzugsweise als abnehmbares Gerät z.B. an den Druckluftanschluß (20) an den Druckapplikator anschließbar sein kann.

Bei Implantation in primärer Zementiertechnik muß ein anderer Druck-/Volumenverlauf realisiert werden (Abb.1). Nach einer Füllphase des Femurs mit einem niedrigen Fülldruck muß der Zementdruck kurzfristig erhöht werden, so daß ein bestimmtes Volumen (ca. 6 ml, s.o.) in kurzer Zeit in die Spongiosa eingedrückt werden kann.

In einer bevorzugten Ausführung (Abb.4) wird dazu der Druckminderer (18) entsprechend dem gewünschten niedrigen Fülldruck montiert oder auf den gewünschten Druck umgeschaltet. Die Druckminderer (32) können verbleiben, sofern sie keine weitere Druckreduktion bewirken. Bei der Zementfüllung des coxalen Femurendes von proximal nach distal ("anterograde Zementeinfüllung") sollte ein Absaugschlauch in das Femur bis zum distalen Markraumstopper gelegt werden, der das Femur nach distal hin abdichtet und der bei jeder derartigen Implantation verwendet werden sollte. Nach vollständiger Auffüllung des Femurs mit dem niedrigen Fülldruck wird jetzt der Handhebel (33) an den Handgriff herangezogen. Durch die Stange (25) und den Klemmring (28) wird jetzt die Kolbenstange (25) nach vorne bewegt. Durch entsprechende Wahl der Hebel sollte durch das einmalige Herandrücken von Hebel (33) an den Handgriff die gewünschte Menge von von ca. 6 ml appliziert werden können. Zur Kontrolle des dabei enstehenden Zementdruckes kann z.B. in der Stange (25) ein Kraftmesser (z.B. Druckfeder) integriert sein, der (bzw. die) über eine entsprechende Skalierung und Eichung ein Ablesen des applizierten Zementdruckes ermöglicht. Im Bereich des Klemmringes (28) kann die Kolbenstange (14) aufgerauht oder oberflächenstruktruriert sein, so daß das Verklemmen des Klemmringes erleichtert wird.

Auch andere Realisierungen des erneuten Ansteigens des Zementapplikationsdruckes sind denkbar. Z.B. kann im Fall einer direkten Ankopplung des Druckluftschlauches an den Druckapplikator ein Handschalter vorgesehen werden, der zwei Funktionen erfüllt: Bei Betätigen des Handschalters erhöht erstens der Druckminderer zwischen Druckluftschlauch und Druckapplikator den Luftdruck zum Erreichen des gewünschten hohen Zementdruckes. Zum zweiten wird eine Markierung an der Kolbenstange (14) oder an einer damit verbundenen Struktur festgeklemmt, die nach Applikation einer Erstmenge von in der Regel 6 ml Knochenzement an einem Schalter vorbeifährt, der den Druckminderer zwischen Druckluftschlauch und Druckapplikator betätigt und den Luftdruck erniedrigt bzw die Druckluft komplett abläßt.

Es ist aber auch möglich, ein zusätzliches Luftdruckreservoir vorzusehen, das ebenfalls über einen Handschalter geöffnet werden kann, so daß sich der auf den Kolben (13) wirkende Druck erhöht. Die Reduktion des Druckes nach der Applikation von 6 ml Knochenzement kann dann wie oben beschrieben erfolgen, indem ein entprechender Druckminderer im Luftdruckreservoir betätigt wird.

Es ist ebenfalls möglich, durch Blockade der Entlüftung (34) einen druckmindergeregelten Gegendruck zwischen Kolben (13) und einer entsprechend abgedichteten Anschlagsperre (17) aufzubauen und bei Betätigen des Handschalters über einen Druckminderer abzulassen. Die Reduktion des Druckes nach der Applikation von 6 ml Knochenzement kann dann wie oben beschrieben erfolgen, indem ein entprechender Druckminderer im Luftdruckreservoir betätigt wird.

Zur besseren Kontrolle des bereits applizierten Zementes kann eine Skalierung (29) am Kolben (13) oder an der Kolbenstange (14) oder einer damit verbundenen Struktur so angebracht sein, daß sie - z.B. durch den Schlitz im Zementspritzenaufnehmer (26) - einsehbar ist und die Relativbewegung zwischen der Kolbenbewegung (13) und Gehäuse des Druckapplikators (35) und so über eine entsprechende Eichung die Menge des applizierten Zementes anzeigt.

Alle Teile der Mischvorrichtung und des Druckapplikators können aus Metall oder Kunststoffen gefertigt sein, von denen sich der Knochenzement leicht ablösen läßt, was eine Wiederverwendung der Teile zuläßt. Dazu müssen diese Teile so gefertigt sein. daß ein hinterschneidungsfreies Herausschlagen des Zementes aus dem Zylinder (1) und aus den Zementapplikationsaufsätzen möglich ist. In der bevorzugten Ausführung des Mischzylinders (1) kann z.B. der Zylinder nach Abnahme des Überwurfs (6) in den Ausschlagring (8) gestülpt werden. Der Stempel (9) oder ein anderes geeignetes Gerät kann auf den Kolben (2) aufgesetzt und der augehärtete Zement aus dem Zylinder (1) ausgeschlagen werden. Auf die gleiche Weise kann auch der Zement aus dem Zementapplkationsaufsatz ausgeschlagen werden, wobei als Ausschlagring z.B. der Zylinder (1) oder aber ein spezieller Ausschlagring verwendet werden kann.

Die folgenden Abbildungen dienen der Erläuterungen einiger bevorzugter Konstruktionsbeispiele und Funktionsprinzipien. Es zeigen:
- Abb.1: beispielhaft die Druck-/Volumencharakteristik für die Zementeinbringung in das coxale Femurende (bei der Implantation eines Hüftschaftes) für die primäre und sekundäre (Zementkanalprothese) Zementiertechnik. Die angegebenen Drucke stellen in etwa den jetzigen Stand der Implantationstechnik dar, die Druckerhöhung auf 3 bar bei der primären Zementiertechnik ist nur als Anhaltswert zu verstehen.

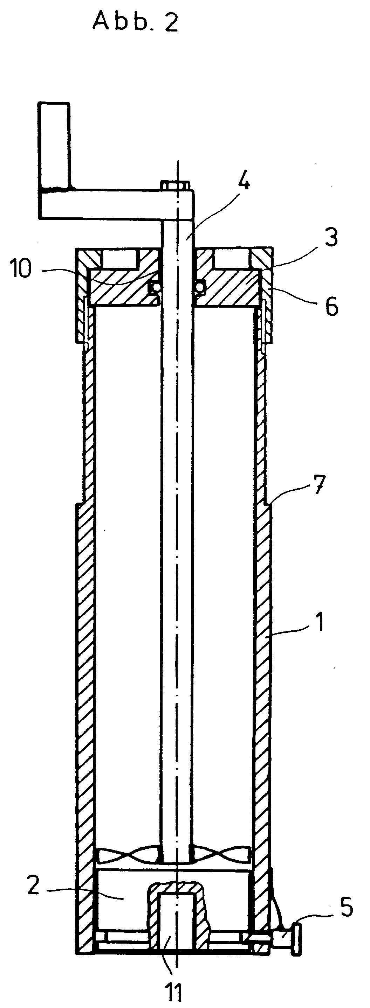

- Abb.2: eine Darstellung des Mischgefäßes, dessen Wandung aus dem Zylinder (1), dem Kolben (2) und dem Mischaufsatz (3) bestehen. Dabei ist der ist der Mischstab (4) Teil des Mischaufsatzes, wobei beide Teile direkt auf den Mischzylinder und nicht auf ein zusätzliches Vakuumgefäß aufgesetzt werden. Wesentliche Aufgabe des Mischstabes ist das Herunterdrücken des Kolbens (2) zum Erzeugen eines Vakuums im Mischgefäß.

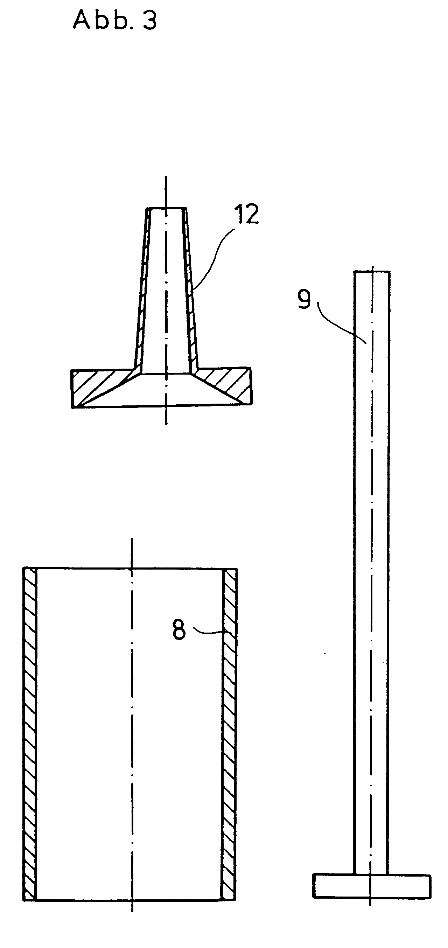

- Abb.3: Beispiele für einen zur Implantation einer Zementkanalprothese geeigneten Zementapplikationsaufsatz (12), einen zum Ausschlagen des ausgehärteten Knochenzementes aus dem Mischzylinder (1) geeigneten Ausschlagring (8) sowie einen zum Ausdrücken des weichen Zementes bei der Implantation bzw. zum Ausschlagen des ausgehärteten Zementes aus dem Mischzylinder (1) geeigneten Stempel (9).
- Abb.4: eine bevorzugte Ausführung eines volumen- und druckgesteuerten Druckapplikators (35). In dieser Ausführung erfolgt die volumengesteuerte Druckreduzierung durch den Druckabfall im Luftreservoir (15) durch den Hauptdruckbegrenzer (18) sowie die Druckminderer (32), die nach Vorbeifahren des Kolben (13) an diesen Druckminderen den Druck im Luftreservoir begrenzen. Die bei der primären Zementierung vorteilhafte Druckerhöhung nach Auffüllung der Markhöhle wird in dieser Ausführung durch den Vorschubmechanismus erzielt, der aus dem Hebel (33), der Stange (25) und dem Klemmring (28) besteht. Z.B. in der Stange (25) kann vorteilhaft ein Kraftmesser integriert sein, der bei entsprechender Eichung den im Zylinder (1) herrschenden Zementdruck anzeigt. Der Druckmesser (19) erlaubt ein Ablesen des im Luftreservoir herrschenden Luftdruckes bzw. - bei entsprechender Eichung - des im Zylinder (1) herrschenden Zementdruckes.
- Abb.5: eine bevorzugte Ausführung eines Druckapplikators mit integrierter Handpumpe, so daß der im Luftreservoir (15) herrschende Druck notfalls ohne von außen zugeführte Druckluft erzeugt werden kann.

## Patentansprüche

1. Verfahren zum Mischen von Knochenzement im Vakuum, dadurch gekennzeichnet, dass das Vakuum durch Volumenänderung eines den Knochenzement aufnehmenden Mischbechers erzeugt wird.

2. Verfahren nach Anspruch 1, dadurch gekennzeichnet, dass das Volumen eines den Mischbecher umschließenden Gefäßes verändert wird.

3. Vorrichtung zum Mischen von Knochenzement mit einem zylindrischen Mischbecher (1), der als Boden einen Kolben (2) und an einem Ende eine Einrastverschlussvorrichtung (5) hat, mit der der Kolben (2) an dem einen Ende des Mischbechers verriegelt und entriegelt werden kann, sowie ferner mit einem Mischaufsatz (3), der auf das andere Ende des zylindrischen Mischbechers aufgesetzt werden kann, wobei der Mischbecher (1) einen Mischstab (4) zum Vermischen der Einzelkomponenten des Knochenzementes aufweist und der Mischstab (4) längsverschieblich in dem Mischaufsatz (3) oder dem Kolben luftdicht gelagert ist, dadurch gekennzeichnet, dass der Mischaufsatz (3) gegenüber dem Mischbecher (1) luftdicht abgedichtet ist, der Kolben (2) nach Einfüllen der Komponenten des Knochenzementes aus seiner verriegelten Stellung in eine zweite Stellung verschiebbar ist und der Mischstab (4) den Kolben (2) nach luftdichtem Verschliessen des Mischbechers (1) mittels des Mischaufsatzes (2) wieder in die verriegelte Stellung verschieben kann.

4. Vorrichtung nach Anspruch 3, dadurch gekennzeichnet, dass zum Verschieben des Kolbens aus der verriegelten in die entriegelte Stellung eine auf den dem Mischstab (4) abgewandten Boden des Kolbens (2) wirkende Stempelstange (9) vorgesehen ist.

5. Vorrichtung nach Anspruch 3 oder 4, dadurch gekennzeichnet, dass eine Einrichtung (9) vorgesehen ist, die es ermöglicht, vor dem luftdichten Aufsetzen des Mischaufsatzes (3) den Kolben (2) in Richtung auf den Mischaufsatz zu bewegen, bis die Komponenten des Knochenzementes mit einer dem Mischaufsatz zugewandten Marke des zylindrischen Gehäuses abschließen.

6. Vorrichtung gemäß einem der Ansprüche 3 bis 5, dadurch gekennzeichnet, dass der Mischstab (4) über mindestens einen Flügel verfügt.

7. Vorrichtung gemäß Anspruch 6, dadurch gekennzeichnet, dass der Mischstab (4) als Rührstab mit propellerartigen Flügeln ausgestattet ist, die beim Drehen des Rührstabes für eine gerichtete Durchströmung des Knochenzementes sorgen.

8. Vorrichtung gemäß Anspruch 7, dadurch gekennzeichnet, dass der Mischstab (4) so geformt ist, dass bei Rotation die peripheren Zementanteile nach unten und die zentralen Zementanteile nach oben gedrückt werden oder umgekehrt, um ein Aufsteigen etwaiger in dem Knochenzement befindlicher Luftblasen an die Oberfläche zu gewährleisten.

9. Vorrichtung nach einem der Ansprüche 3 bis 8, dadurch gekennzeichnet, dass sie verschiedene Applikationsaufsätze (12) zur Implantation in anterograder oder retrograder Implantationstechnik oder zur Implantation einer Zementkanalprothese aufweist.

10. Vorrichtung nach Anspruch 9, dadurch gekennzeichnet, dass die Applikationsaufsätze (12) wie auch der Mischaufsatz (3) mit einer gemeinsamen Überwurfvorrichtung (6) am Mischzylinder befestigt sind.

11. Vorrichtung nach Anspruch 4, dadurch gekennzeichnet, dass der Kolben (2) über eine Aussparung (11) verfügt, in die die Stempelstange (9) mit geringem Spiel sowie eine Kolbenstange (14) mit viel Spiel hineinpaßt, so dass der Kolben (2) mit Hilfe dieser Teile von Hand in Richtung auf den Mischaufsatz bewegt werden kann und auch im Sinne einer herkommlichen Spritze oder durch Verwendung eines Druckapplikators (35) in Richtung auf den Mischaufsatz zum Ausdrücken des Zementes bewegt werden kann.

12. Vorrichtung nach einem der Ansprüche 3 bis 11, dadurch gekennzeichnet, dass eine Ringhalterung (8) den Mischzylinder nach Applikation, Aushärten des Zementes und Abnehmen des Überwurfes (6) so aufnimmt, dass er an einer Nase (7) aufsitzt und der Zement durch Vortreiben des Kolben mit der Stempelstange (9) ausgeschlagen werden kann.

13. Vorrichtung nach einem der Ansprüche 3 bis 12, dadurch gekennzeichnet, dass Mischzylinder (1), Applikationsaufsätze (12) und Überwurf (6) so hinterschneidungsfrei konstruiert sind, dass der ausgehärtete Zement in mindestens eine Richtung ausgeschlagen werden kann.

14. Vorrichtung nach einem der Ansprüche 3 bis 13, dadurch gekennzeichnet, dass mit dem Mischbecher (1) ein Druckapplikator (35) und eine Zementspritze verbunden sind, und dass der Druckapplikator eine Einrichtung zum Regeln des Druckes für die Zementspritze aufweist, so dass eine volumengesteuerte Austreibung des Zementes mit vorgebbaren Drucken ermöglicht wird.

15. Vorrichtung nach Anspruch 14, dadurch gekennzeichnet, dass ein Kolben (13) des Druckapplikators (35) in seiner Ausgangposition reversibel mit einer entsprechenden Vorrichtung (16) des Druckapplikators verriegelbar ist und nach seiner Entriegelung auf die Zementspritze einwirkt und den Zement ausdrückt, wobei der Druckapplikator (35) ein Luftreservoir (15) aufweist, und der Kolben (13) von der Druckluft des Luftreservoirs (15) angetrieben wird und der Druck in dem Luftreservoir durch einen Druckbegrenzer zunächst initial in Richtung auf den Mischbecher begrenzt und durch eine Rückkopplung anschließend abgesenkt wird, wobei der Kolbenweg des Kolben (13) als Maß des ausgedrückten Zementvolumens das Maß der Druckminderung im Sinne der Rückkopplung vorgibt.

16. Vorrichtung nach Anspruch 14 oder 15, dadurch gekennzeichnet, dass der Druckapplikator (35) hydraulisch angetrieben wird.

17. Vorrichtung nach Anspruch 14 oder 15, dadurch gekennzeichnet, dass der Druckapplikator (35) elektrisch oder mechanisch (Handkraft) angetrieben wird.

18. Vorrichtung nach Anspruch 15, dadurch gekennzeichnet, dass die Rückkopplung durch Schalter bewirkt wird, die von dem Kolben (13), der Kolbenstange (14) oder von einer mit diesen Teilen verbundenen Struktur des Druckapplikators (35) ausgelöst werden und mechanisch, hydraulisch oder elektrisch eine Verstellung des Druckbegrenzers bewirken.

19. Vorrichtung nach Anspruch 15, dadurch gekennzeichnet, dass die im Luftreservoir druckreduzierende Wirkung im Sinne der Rückkopplung durch weitere Druckbegrenzer bewirkt wird, die direkt an der Zylinderwand (36) angebracht sind, so dass es nach dem Vorbeifahren des Kolbens an einem bestimmten Druckminderer zu einem Kontakt dieses Druckminderers zu dem Luftreservoir und so zu einem Druckabfall in dem Luftreservoir kommt.

20. Vorrichtung nach einem der Ansprüche 15 und 19, dadurch gekennzeichnet, dass die Druckminderer aus Federzungen (30) bestehen, die ein Dichtelement (31) von außen mit einer durch die Abmessungen der Federzunge bestimmten Kraft auf die Bohrungen in der Zylinderwand des Druckkolbens drücken, so dass sich aus der Kraft der Federzunge und der Fläche der Bohrung der Druck bestimmt, der maximal in dem Zylinder herrschen kann.

21. Vorrichtung nach Anspruch 15, dadurch gekennzeichnet, dass der Druck im Luftreservoir über einen Anschluss an die OP-Druckluftquelle aufgebaut werden kann .

22. Vorrichtung gemäß einem der Ansprüche 14 bis 21, dadurch gekennzeichnet, dass beim Herunterdrücken des Kolben (13) in seine Ausgangposition der hinter dem Kolben befindliche Luftdruck mit Hilfe eines Ventils (21) abgelassen werden kann.

23. Vorrichtung gemäß Anspruch 22, dadurch gekennzeichnet, dass eine Entlüftungsbohrung (34) den Aufbau eines Gegendruckes auf der dem Ventil (21) gegenüberliegenden Seite des Kolbens (13) verhindert.

24. Vorrichtung gemäß Anspruch 14, dadurch gekennzeichnet, dass der Mischbecher (1) mit dem Druckapplikator über ein längsverschiebliches Schienensystem in der Stellung verbindbar ist, in der der Zement gerade an dem Applikationsaufsatz (5) beginnt auszutreten.

25. Vorrichtung gemäß Anspruch 24, dadurch gekennzeichnet, dass der Mischbecher (1) in einem an dem Druckapplikator befestigten geschlitzten Zylinder (26) eingeführt wird und längsverschieblich ist und dort in der gewünschten Stellung mit Hilfe einer Haltevorrichtung fixierbar ist.

26. Vorrichtung gemäß einem der Ansprüche 14 bis 16, dadurch gekennzeichnet, dass das Luftreservoir (15) mit einer fest im Druckapplikator (35) integrierten (22) oder einer aufsetzbaren Handpumpe verbunden ist, mit der ein Druck von mindestens 8 bar erzeugbar ist.

27. Vorrichtung gemäß einem der Ansprüche 14 bis 16, dadurch gekennzeichnet, dass das Luftreservoir (15) bzw. der Raum direkt vor dem Kolben (13) mit einem Druckmesser (19) versehen ist.

28. Vorrichtung gemäß einem der Ansprüche 14 bis 27, dadurch gekennzeichnet, dass für den Kolben (13) eine Anschlagsperre (17) vorgesehen ist, die ein unkontrolliertes Herausschießen des Kolbens verhindert.

29. Vorrichtung gemäß einem der Ansprüche 14 bis 28, dadurch gekennzeichnet, dass eine Skalierung (29) so mit dem Kolben (13) oder der Kolbenstange (14) oder mit einer diesen Teilen anhaftenden Struktur verbunden ist, dass deren Relativbewegung zu dem Gehäuse des Druckapplikators gemessen und bei entsprechender Eichung die Menge des applizierten Zementes abgelesen werden kann.

30. Vorrichtung nach einem der Ansprüche 3 bis 29, dadurch gekennzeichnet, dass der Mischstab (4) in dem Kolben (2) des Michbechers gelagert und mit diesem verriegelbar ist.

31. Vorrichtung nach einem der Ansprüche 3 bis 30, dadurch gekennzeichnet, dass der Applikationsaufsatz für eine Implantation in primärer Zementiertechnik aus einem sich an die Form der Knochenöffnung anpassenden Material besteht.

## Claims

1. Process for mixing bone cement *in vacuo*, characterised in that the vacuum is generated by altering the volume of a mixing beaker receiving the bone cement.

2. Process according to claim 1, characterised in that the volume of a vessel surrounding the mixing beaker is altered.

3. Apparatus for mixing bone cement, having a cylindrical mixing beaker (1) which has a piston (2) as its bottom and at one end a snap-in closure device (5) with which the piston (2) can be locked and unlocked at the one end of the mixing beaker, and also having a mixing attachment (3) which can be placed onto the other end of the cylindrical mixing beaker, the mixing beaker (1) featuring a mixing rod (4) for blending the individual components of the bone cement and the mixing rod (4) being mounted in airtight manner so as to be longitudinally displaceable in the mixing attachment (3) or the piston, characterised in that the mixing attachment (3) is sealed in airtight manner with respect to the mixing beaker (1), after filling with the components of the bone cement the piston (2) can be moved from its locked position into a second position, and the mixing rod (4) can move the piston (2) back into the locked position once the mixing beaker (1) has been closed in airtight manner by means of the mixing attachment (2).

4. Apparatus according to claim 3, characterised in that a plunger rod (9) which acts on the bottom of the piston (2) remote from the mixing rod (4) is provided for moving the piston out of the locked position and into the unlocked position.

5. Apparatus according to claim 3 or 4, characterised by the provision of a device (9) which makes it possible to move the piston (2) towards the mixing attachment before the mixing attachment (3) is placed on top in airtight manner, until the components of the bone cement close with a mark on the cylindrical vessel, which mark faces the mixing attachment.

6. Apparatus according to any of claims 3 to 5, characterised in that the mixing rod (4) is provided with at least one vane.

7. Apparatus according to claim 6, characterised in that the mixing rod (4) is a stirrer rod equipped with propeller-like vanes, which ensure a directed throughflow of the bone cement as the stirrer rod turns.

8. Apparatus according to claim 7, characterised in that the mixing rod (4) is so formed that upon rotation the peripheral cement constituents are forced downwards and the central cement constituents upwards or *vice versa*, in order to make sure that any air bubbles in the bone cement rise to the surface.

9. Apparatus according to any of claims 3 to 8, characterised in that it has various application attachments (12) for implantation in anterograde or retrograde implantation technology or for implanting a cement canal prosthesis.

10. Apparatus according to claim 9, characterised in that both the application attachments (12) and the mixing attachment (3) are fastened to the mixing cylinder with a common retaining fixture (6).

11. Apparatus according to claim 4, characterised in that the piston (2) is provided with a recess (11) into which the plunger rod (9) fits with little freeplay as well as a piston rod (14) with a large amount of freeplay, with the result that the piston (2) can be moved by hand with the help of these parts towards the mixing attachment and can also be moved in the manner of a conventional syringe or by the use of a pressure applicator (35) towards the mixing attachment to force out the cement.

12. Apparatus according to any of claims 3 to 11, characterised in that following application and hardening of the cement and removal of the retainer (6) an annular mount (8) receives the mixing cylinder in such a way that the latter sits on a lug (7) and the cement can be knocked out by driving the piston forward with the plunger rod (9).

13. Apparatus according to any of claims 3 to 12, characterised in that the mixing cylinder (1), application attachments (12) and retainer (6) are designed without any undercuts so that the hardened cement can be knocked out in at least one direction.

14. Apparatus according to any of claims 3 to 13, characterised in that connected to the mixing beaker (1) are a pressure applicator (35) and a cement syringe, and that the pressure applicator incorporates a device for regulating the pressure in respect of the cement syringe, thereby making it possible to expel a controlled volume of the cement at pre-specifiable pressures.

15. Apparatus according to claim 14, characterised in that a piston (13) of the pressure applicator (35) is adapted to be locked in its initial position reversibly with a corresponding fixture (16) of the pressure applicator and after being unlocked it operates on the cement syringe and forces out the cement; the pressure applicator (35) incorporates an air reservoir (15) and the piston (13) is driven by the compressed air of the air reservoir (15), and the pressure in the air reservoir is at first limited by a pressure controller initially in the direction towards the mixing beaker and later lowered by feedback; the distance travelled by the piston (13), being a measure of the volume of cement expelled, specifies the extent of the pressure decrease due to the feedback.

16. Apparatus according to claim 14 or 15, characterised in that the pressure applicator (35) is driven hydraulically.

17. Apparatus according to claim 14 or 15, characterised in that the pressure applicator (35) is driven electrically or mechanically (by manual force).

18. Apparatus according to claim 15, characterised in that the feedback is effected by switches which are triggered by the piston (13), the piston rod (14) or by a pressure applicator (35) structure connected to these parts and brings about an adjustment of the pressure controller mechanically, hydraulically or electrically.

19. Apparatus according to claim 15, characterised in that the pressure-reducing action in the air reservoir due to the feedback is brought about by additional pressure controllers which are mounted directly on the cylinder wall (36), with the result that once the piston has travelled past a given pressure reducer, contact is made between this pressure reducer and the air reservoir and thus a fall in pressure ensues in the air reservoir.

20. Apparatus according to either of claims 15 and 19, characterised in that the pressure reducers consist of flexible tongues (30) which from the outside press a sealing element (31) onto the bores in the cylinder wall of the pressure piston with a force determined by the dimensions of the flexible tongue, with the result that the force of the flexible tongue and surface of the bore determine the maximum possible pressure in the cylinder.

21. Apparatus according to claim 15, characterised in that the pressure in the air reservoir can be built up via a connection to the operating theatre's compressed air source.

22. Apparatus according to any of claims 14 to 21, characterised in that as the piston (13) is being pushed down into its initial position the air pressure behind the piston can be relieved with the help of a valve (21).

23. Apparatus according to claim 22, characterised in that an air vent (34) prevents the build-up of a counterpressure on the opposite side of the piston (13) to the valve (21).

24. Apparatus according to claim 14, characterised in that the mixing beaker (1) is adapted to be connected to the pressure applicator via a longitudinally displaceable runner system, in the position in which the cement just begins to emerge from the application attachment (5).

25. Apparatus according to claim 24, characterised in that the mixing beaker (1) is set in a slotted cylinder (26) fastened to the pressure applicator and is longitudinally displaceable and with the help of a holder can be fixed there in the desired position.

26. Apparatus according to any of claims 14 to 16, characterised in that the air reservoir (15) is connected to a hand pump which is permanently integrated (22) in the pressure applicator (35) or else attachable to it, and with which a pressure of at least 8 bar can be generated.

27. Apparatus according to any of claims 14 to 16, characterised in that the air reservoir (15), or rather the space directly ahead of the piston (13), is provided with a pressure gauge (19).

28. Apparatus according to any of claims 14 to 27, characterised in that for the piston (13) a limit stop (17) is provided which prevents the piston from shooting out uncontrolledly.

29. Apparatus according to any of claims 14 to 28, characterised in that a scale (29) is connected to the piston (13) or to the piston rod (14) or to a structure adhering to one of these parts, such that the movement thereof relative to the pressure applicator casing can be measured and, given suitable calibration, the quantity of cement applied can be read off.

30. Apparatus according to any of claims 3 to 29, characterised in that the mixing rod (4) is mounted in the piston (2) of the mixing beaker and adapted to be locked thereto.

31. Apparatus according to any of claims 3 to 30, characterised in that the application attachment for an implantation in primary cementation technology is of a material that moulds itself to the shape of the opening in the bone.

## Revendications

1. Procédé pour mélanger un ciment à os sous vide, caractérisé en ce que le vide est obtenu par modification de volume d'un godet contenant le ciment à os.

2. Procédé selon la revendication 1, caractérisé en ce qu'on modifie le volume d'un récipient renfermant le godet.

3. Dispositif pour mélanger un ciment à os à l'aide d'un godet cylindrique (1) comprenant, au fond, un piston (2) et, à une extrémité, un dispositif de verrouillage par enclenchement (5) à l'aide duquel on peut verrouiller et déverrouiller le piston (2) à l'extrémité du godet, comprenant en outre une garniture de mélange (3) qui peut être montée à l'autre extrémité du godet cylindrique, le godet de mélange (1) étant muni d'une tige de mélange (4) pour mélanger les composants individuels du ciment à os et la tige de mélange (4) étant montée à coulissement longitudinal et de manière étanche dans la garniture de mélange (3) ou dans le piston, caractérisé en ce que la garniture de mélange (3) est rendue étanche à l'air par rapport au godet de mélange (1), et en ce que le piston (2), après remplissage avec les composants du ciment à os, est susceptible de coulisser de sa position verrouillée dans une deuxième position et en ce que la tige de mélange (4) est susceptible de faire coulisser le piston (2) de nouveau dans sa position de verrouillage après la fermeture étanche à l'air du godet de mélange (1) au moyen de la garniture de mélange (3).

4. Dispositif selon la revendication 3, caractérisé en ce que, pour faire coulisser le piston de la position verrouillée à la position déverrouillée, on prévoit un poussoir (9) agissant sur le fond du piston (2) du côté opposé à la tige de mélange (4).

5. Dispositif selon la revendication 3 ou 4, caractérisé en ce qu'il comprend un dispositif (9) qui permet de déplacer le piston (2) dans la direction de la garniture de mélange (3) avant d'installer celle-ci de manière étanche à l'air, jusqu'à ce que les composants du ciment à os atteignent une marque du boîtier cylindrique du côté de la garniture de mélange.

6. Dispositif selon l'une quelconque des revendications 3 à 5, caractérisé en ce que la tige de mélange (4) est raccordée à au moins une pale.

7. Dispositif selon la revendication 6, caractérisé en ce que la tige de mélange (4) est munie de pales formant des hélices qui, lors d'une rotation de la tige de mélange, assurent un écoulement dirigé du ciment à os.

8. Dispositif selon la revendication 7, caractérisé en ce que la tige de mélange (4) est de forme telle que, lors d'une rotation, les parties de ciment périphériques sont poussées vers le bas et les parties de ciment centrales sont poussées vers le haut, ou inversement, pour entraîner l'échappement à la surface d'éventuelles bulles d'air se trouvant dans le ciment à os.

9. Dispositif selon l'une quelconque des revendications 3 à 8, caractérisé en ce qu'il comprend divers applicateurs (12) afin de réaliser une implantation selon la technique antérograde ou rétrograde, ou pour implanter une prothèse à canal en ciment.

10. Dispositif selon la revendication 9, caractérisé en ce que les applicateurs (12) ainsi que la garniture de mélange (3) sont fixés au cylindre de mélange par une bague commune (6).

11. Dispositif selon la revendication 4, caractérisé en ce que le piston (2) est muni d'un évidement (11) dans lequel pénètre le poussoir (9) avec faible jeu ainsi qu'une tige de piston (14) avec un jeu important, de manière que le piston (2) puisse être déplacé vers la garniture de mélange à la main à l'aide de ces éléments, et puisse également être déplacé, à la façon d'une seringue classique ou en utilisant un applicateur de pression (35), dans la direction de la garniture de mélange pour expulser le ciment.

12. Dispositif selon l'une quelconque des revendications 3 à 11, caractérisé en ce qu'un support annulaire (8) entoure le cylindre de mélange après l'application et le durcissement du ciment et le retrait de la bague (6), de manière qu'il repose sur un épaulement (7) et que le ciment puisse être extrait en repoussant le piston avec le pousseur (9).

13. Dispositif selon l'une quelconque des revendications 3 à 12, caractérisé en ce que le cylindre de mélange (1), les applicateurs (12) et la bague (6) sont réalisés sans contre-dépouille de manière que le ciment durci puisse être expulsé dans au moins un sens.

14. Dispositif selon l'une quelconque des revendications 3 à 13, caractérisé en ce qu'un applicateur de pression (35) et une seringue à ciment sont associés au godet de mélange (1) et en ce que l'applicateur de pression est muni d'un dispositif pour régler la pression de la seringue à ciment de manière à permettre une expulsion du ciment commandée en volume et à pression choisie.

15. Dispositif selon la revendication 14, caractérisé en ce qu'un piston (13) de l'applicateur de pression (35) est verrouillé de manière réversible dans sa position de sortie avec un dispositif (16) correspondant de l'applicateur de pression et agit après son déverrouillage sur la seringue à ciment et expulse le ciment, l'applicateur de pression (35) étant muni d'un réservoir d'air (15), et le piston (13) étant entraîné par l'air sous pression du réservoir d'air (15), et la pression dans le réservoir d'air étant d'abord limitée par un limiteur de pression dans la direction du godet de mélange et ensuite abaissée par un retour, d'où il résulte que la course du piston (13), en tant que mesure du volume de ciment expulsé, donne d'avance la mesure de la diminution de la pression dans le retour.

16. Dispositif selon la revendication 14 ou 15, caractérisé en ce que l'applicateur de pression (35) est commandé de manière hydraulique.

17. Dispositif selon la revendication 14 ou 15, caractérisé en ce que l'applicateur de pression (35) est commandé de manière électrique ou mécanique (à la main).

18. Dispositif selon la revendication 15, caractérisé en ce que le retour est réalisé par des commutateurs qui sont actionnés par le piston (13), par la tige de piston (14) ou par une structure de l'applicateur de pression (35) reliée à l'une de ces pièces, et opère un réglage du limiteur de pression de manière mécanique, hydraulique ou électrique.

19. Dispositif selon la revendication 15, caractérisé en ce que l'action de réduction de pression dans le réservoir d'air du fait du retour est réalisée par des limiteurs de pression supplémentaires qui sont directement rapportés sur la paroi cylindrique (36), de manière que, lors du passage du piston au niveau d'un réducteur de pression déterminé, il naît une communication entre ce réducteur de pression et le réservoir d'air et donc une diminution de pression dans le réservoir d'air.

20. Dispositif selon l'une quelconque des revendications 15 à 19, caractérisé en ce que les réducteurs de pression sont à languettes élastiques (30) qui sollicitent des éléments d'obturation (31) depuis l'extérieur, avec une force déterminée par les dimensions des languettes élastiques, sur des perçages dans la paroi cylindrique de l'applicateur de pression, de manière à déterminer la pression maximale qui peut régner dans le cylindre en fonction de la force des languettes élastiques et les sections des perçages.

21. Dispositif selon la revendication 15, caractérisé en ce que la pression dans le réservoir d'air peut être établie par un branchement à une source d'air sous pression.

22. Dispositif selon l'une quelconque des revendications 14 à 21, caractérisé en ce que, lors de la poussée du piston (13) jusqu'à sa position de sortie, la pression d'air à l'arrière du piston peut être évacuée par une soupape (21).

23. Dispositif selon la revendication 22, caractérisé en ce qu'un perçage d'échappement (34) empêche l'établissement d'une contre-pression du côté du piston (13) à l'opposé de la soupape (21).

24. Dispositif selon la revendication 14, caractérisé en ce que le godet de mélange (1) peut être relié à l'applicateur de pression par un système de rail à coulissement longitudinal dans la position à laquelle le ciment commence tout juste à sortir de l'applicateur (5).

25. Dispositif selon la revendication 24, caractérisé en ce que le godet de mélange (1) est inséré à coulissement longitudinal dans un cylindre fendu (26) fixé à l'applicateur de pression, où il est fixé à la position souhaitée à l'aide d'un dispositif de maintien.

26. Dispositif selon l'une quelconque des revendications 14 à 16, caractérisé en ce que le réservoir d'air (15) est relié à une pompe manuelle intégrée de manière fixe (22) dans l'applicateur de pression (35) ou bien amovible, à l'aide de laquelle on peut obtenir une pression d'au moins 8 bars.

27. Dispositif selon l'une quelconque des revendications 14 à 16, caractérisé en ce que le réservoir d'air (15), ou bien l'espace directement devant le piston (13), est muni d'un indicateur de pression (19).

28. Dispositif selon l'une quelconque des revendications 14 à 27, caractérisé en ce qu'il comprend, pour le piston (13), une butée (17) empêchant une projection en dehors incontrôlée du piston.

29. Dispositif selon l'une quelconque des revendications 14 à 28, caractérisé en ce qu'une graduation (29) est rapportée sur le piston (13) ou la tige de piston (14) ou sur une structure reliée à ces éléments, de manière que son mouvement relatif par rapport au boîtier de l'applicateur de pression puisse être mesuré et que l'on puisse relever, avec un étalonnage correspondant, la quantité de ciment appliquée.

30. Dispositif selon l'une quelconque des revendications 3 à 29, caractérisé en ce que la tige de mélange (4) est logée dans le piston (2) du godet de mélange et peut être verrouillée par rapport à celui-ci.

31. Dispositif selon l'une quelconque des revendications 3 à 30, caractérisé en ce que l'applicateur est en un matériau adapté à la forme de l'ouverture de l'os pour une implantation selon une technique primaire d'application de ciment.
